# EUROPÄISCHE PATENTSCHRIFT

(11) **EP 3 411 127 B1**
(45) Veröffentlichungstag und Bekanntmachung des Hinweises auf die Patenterteilung: **16.10.2019**
(21) Anmeldenummer: 17702128.4
(22) Anmeldetag: 02.02.2017
(51) Int. Cl.: B01D 3/00, B01D 3/14, B01D 3/32, B01D 3/40, C07C 7/00, C07C 7/08, C07C 9/10, C07C 11/08, C01B 7/07, C07C 29/84, C07C 67/54, C07C 45/83, C07C 253/34, C07C 11/16, C07D 307/08, C07C 17/386

(54) **VERFAHREN ZUR TRENNUNG VON STOFFEN DURCH EXTRAKTIVDESTILLATION**
METHOD FOR SEPARATING MATERIALS BY MEANS OF AN EXTRACTIVE DISTILLATION PROCESS
PROCÉDÉ DE SÉPARATION DE SUBSTANCES PAR DISTILLATION EXTRACTIVE

(30) Priorität: 05.02.2016 EP 16154499
(43) Veröffentlichungstag der Anmeldung: 12.12.2018
(73) Patentinhaber: BASF SE, 67056 Ludwigshafen am Rhein (DE)
(72) Erfinder: ASPRION, Norbert, 67056 Ludwigshafen (DE)
(74) Vertreter: BASF IP Association
(86) Internationale Anmeldenummer: PCT/EP2017/052215
(87) Internationale Veröffentlichungsnummer: WO 2017/134143

(56) Entgegenhaltungen:
- EP-A1- 0 366 019
- DE-A1- 2 208 195
- US-A- 4 447 318
- US-A1- 2014 124 358

## Beschreibung

Die Erfindung betrifft ein Verfahren zur Trennung von Stoffen durch Extraktivdestillation.

Es ist bekannt, dass sich durch gewöhnliche Destillation nicht oder schwer auftrennbare azeotrope oder engsiedende Gemische häufig durch eine Extraktivdestillation trennen lassen.

Bei der Extraktivdestillation bringt man den Brüden mit einem Extraktionsmittel in Kontakt, das selektiv die Flüchtigkeit einer der Komponenten des zu trennenden Gemischs verringert. Das Extraktionsmittel weist einen höheren Siedepunkt bzw. einen deutlich niedrigeren Dampfdruck auf als die zu trennenden Stoffe. Es wird zusammen mit einem der zu trennenden Stoffe über den Sumpf der Kolonne abgezogen. Der extrahierte Stoff kann in einem Stripper freigesetzt und das aus dem Sumpf des Strippers abgezogene regenerierte Extraktionsmittel wiederverwendet werden.

Da Verfahren zur Trennung von Stoffen durch Extraktivdestillation mehrere Destillationen umfassen und daher energieintensiv sind, ist eine optimale Energieintegration wesentlich für ihren wirtschaftlichen Betrieb. Es ist bekannt, die thermische Energie des regenerierten Extraktionsmittels zur Beheizung der Extraktivdestillationskolonne bzw. zur Vorerwärmung des beladenen Extraktionsmittelstroms rückzugewinnen. So beschreibt die US 2014/0124358 A1 ein Verfahren, bei dem eine mehrstufige Aufheizung der Extraktivdestillationskolonne über seitlich an der Kolonne angebrachte Verdampfer erfolgt. Jeder Verdampfer erwärmt einen von einem Sammelboden abgezogenen Seitenabzug der Kolonne. Der flüssig/dampfförmig-gemischtphasige Strom aus den Verdampfern wird in die Extraktivdestillationskolonne zurückgeführt. Der Kolonnensumpf wird zusätzlich über einen optionalen Verdampfer beheizt. Die seitlich an der Kolonne angebrachten Verdampfer werden mit heißem regeneriertem Extraktionsmittel betrieben. Der optionale Sumpfverdampfer kann zusätzlich mit Niederdruck-Dampf oder Mitteldruck-Dampf betrieben werden.

Das Verfahren der US 2014/0124358 A1 hat den Nachteil, dass die durch Sammelböden abgegrenzten übereinander angeordneten Kompartimente die Bauhöhe der Extraktivdestillationskolonne vergrößern. So sind mindestens übereinander angeordnete Kompartimente erforderlich, wenn eine Aufheizung der Kolonne über mindestens drei Verdampfer erfolgen soll.

Der Erfindung liegt die Aufgabe zugrunde, ein Extraktivdestillationsverfahren anzugeben, das eine optimale Energieintegration ohne wesentliche Vergrößerung der Bauhöhe der Extraktivdestillationskolonne erlaubt.

Die Aufgabe wird gelöst durch ein Verfahren zur Trennung eines Gemisches von Stoffen A und B durch Extraktivdestillation unter Verwendung eines Extraktionsmittels mit einer höheren Affinität zu B als zu A, wobei man
a) einen A und B enthaltenden Zulaufstrom dem Extraktionsmittel in einer Kolonne entgegenführt, wobei eine A enthaltende Kopffraktion sowie eine B und Extraktionsmittel enthaltende Flüssigfraktion erhalten wird,
b) die Flüssigfraktion auf einem Sammelboden sammelt und in einem ersten indirekten Wärmetauscher erwärmt und teilweise verdampft, den entstehenden Dampf in die Kolonne freisetzt und einen nicht verdampften Anteil der Flüssigfraktion als Sumpffraktion im Sumpf der Kolonne sammelt,
c) die Sumpffraktion nacheinander in einem zweiten indirekten Wärmetauscher und einem dritten indirekten Wärmetauscher erwärmt und teilweise verdampft, wobei der entstehende Dampf mindestens teilweise in die Kolonne freigesetzt wird,
d) die Sumpffraktion in einem Stripper in eine B enthaltende Fraktion und eine Extraktionsmittelfraktion auftrennt,
e) die Extraktionsmittelfraktion als Heizmedium für den zweiten Wärmetauscher nutzt, wobei eine teilweise abgekühlte Extraktionsmittelfraktion erhalten wird, und ein externes Heizmedium für den dritten Wärmetauscher verwendet, und
f) die teilweise abgekühlte Extraktionsmittelfraktion als Heizmedium für den ersten Wärmetauscher nutzt.

Erfindungsgemäß wird die Sumpffraktion nacheinander in einem zweiten indirekten Wärmetauscher und einem dritten indirekten Wärmetauscher erwärmt und teilweise verdampft. Der entstehende Dampf wird mindestens teilweise in die Kolonne freigesetzt, vorzugsweise in das Volumen oberhalb des Kolonnensumpfes. Durch die zweifache Wärmeübertragung, bei der die Sumpffraktion zuerst mittels der vom Stripper stammenden Extraktionsmittelfraktion und dann mittels eines externen Heizmediums erwärmt wird, wird die thermische Energie der regenerierten Extraktionsmittelfraktion optimal genutzt. Das externe Heizmedium liefert lediglich den erforderlichen Energieeintrag, um die Sumpftemperatur vom Temperaturniveau, welches durch Beheizung mit der regenerierten Extraktionsmittelfraktion erreicht wird, auf einen gewünschten Wert anzuheben. Außerdem wird Flüssigfraktion auf einem Sammelboden gesammelt und in einem ersten indirekten Wärmetauscher erwärmt und teilweise verdampft. Bedingt durch die niedrigere Temperatur des internen Stoffstromes im Vergleich zur Sumpftemperatur der Kolonne, kann für die Zwischenerhitzung Wärmeenergie auf einem niedrigen Temperaturniveau eingesetzt werden. Erfindungsgemäß wird teilweise abgekühlte Extraktionsmittelfraktion als Heizmedium für den ersten Wärmetauscher genutzt.

Man führt den A und B enthaltenden Zulaufstrom dem Extraktionsmittel in einer Kolonne entgegen. Den A und B enthaltenden Zulaufstrom führt man in einen unteren Bereich oder einen mittleren Bereich der Kolonne ein. Das Extraktionsmittel führt man am Kopf oder in einen oberen Bereich der Kolonne ein. Das Extraktionsmittel wird flüssig in die Kolonne eingeführt. Der Zulaufstrom kann dampfförmig oder flüssig in die Kolonne eingeführt werden.

Als trennwirksame Einbauten enthält die Kolonne vorzugsweise Böden, Füllkörper und/oder Packungen. Die Kolonne kann z.B. eine oberhalb der Zuführung des A und B enthaltenden Zulaufstroms gelegene Verstärkungszone und unterhalb der Zuführung des Zulaufstroms gelegene Abtriebszone aufweisen.

Es wird eine A enthaltende Kopffraktion sowie eine B und Extraktionsmittel enthaltende Flüssigfraktion erhalten.

In der A enthaltenden Kopffraktion ist der Stoffmengenanteil A/(A+B) höher, vorzugsweise mindestens 1,3 mal höher, insbesondere mindestens 1,5 mal höher, als im A und B enthaltenden Zulaufstrom. Den Stoffmengenanteil A/(A+B) berechnet man, indem man die Stoffmenge A durch die Summe der Stoffmenge A und der Stoffmenge B teilt. Die A enthaltende Kopffraktion wird am Kopf der Kolonne aus der Kolonne geführt.

Die B und Extraktionsmittel enthaltende Flüssigfraktion sammelt man auf einem Sammelboden. Der Sammelboden ist in der Kolonne oberhalb des Kolonnensumpfs angeordnet, beispielsweise zwischen dem Sumpf und der Abtriebszone. Die gesammelte Flüssigfraktion wird in einem ersten indirekten Wärmetauscher erwärmt und teilweise verdampft. Der erste Wärmetauscher ist vorzugsweise außerhalb der Kolonne angeordnet. Man kann die Flüssigfraktion vom Sammelboden abziehen und in den außerhalb der Kolonne angeordneten Wärmetauscher führen.

Den beim Erwärmen im ersten Wärmetauscher entstehenden Dampf setzt man in die Kolonne frei und sammelt einen nicht verdampften Anteil der Flüssigfraktion als Sumpffraktion im Sumpf der Kolonne. Im Allgemeinen wird man einen flüssig/dampfförmiggemischtphasigen Strom aus dem Verdampfer, der das aus dem entstehenden Dampf und dem nicht verdampften Anteil der Flüssigfraktion besteht, unterhalb des Sammelbodens in die Kolonne zurückführen. Man kann den entstehenden Dampf auch vom nicht verdampften Anteil der Flüssigfraktion trennen, z.B. in einem Phasenabscheider, und beide Phasen getrennt in die Kolonne führen.

Im erfindungsgemäßen Verfahren kann jede zur Übertragung von Wärme von einer Flüssigkeit auf eine andere Flüssigkeit geeignete Form von indirektem Wärmetauscher als erster Wärmtauscher eingesetzt werden. Der erste Wärmetauscher kann z.B. ein Fallfilmverdampfer, ein Kesselverdampfer, ein Zwangsumlaufverdampfer oder ein Naturumlaufverdampfer sein. Vorzugsweise ist der erste Wärmetauscher ein Naturumlaufverdampfer.

Das erfindungsgemäße Verfahren hat den Vorteil, dass der erste Wärmetauscher auch bei sehr kleinen Abdampfraten, also auch bei geringer Wärmezufuhr, als Naturumlaufverdampfer, also ohne zusätzlichen Kosten- und Energieaufwand für eine Pumpe, betrieben werden kann. Dies ist auf die im Allgemeinen bestehende Höhendifferenz zwischen dem ersten Wärmetauscher und dem weiter oben gelegenen Sammelboden zurückzuführen, sowie auf den statischen Druck der überstehenden Flüssigkeitssäule. Dadurch kann auch bei geringer Abdampfrate (also auch bei geringer Wärmezufuhr) ein kontinuierliches Durchströmen des ersten Wärmetauschers ohne die Gefahr, dass der Umlauf zum Erliegen kommt, sichergestellt werden. Vorzugsweise ist der erste Wärmetauscher so weit unten außerhalb der Kolonne angeordnet, dass der aus dem ersten Wärmetauscher erhaltene, nicht verdampfte Anteil der Flüssigfraktion unterhalb des Sammelbodens, bevorzugt in einem Bereich von 0,15 m bis 0,70 m unterhalb des Sammelbodens, besonders bevorzugt in einem Bereich von 0,20 m bis 0,60 m unterhalb des Sammelbodens, ganz besonders bevorzugt in einem Bereich von 0,25 m bis 0,50 m unterhalb des Sammelbodens anfällt. Beispielsweise kann der erste Wärmetauscher so weit unten außerhalb der Kolonne angeordnet sein, dass dessen Auslass für den nicht verdampften Anteil der Flüssigfraktion oder für den flüssig/dampfförmiggemischtphasigen Strom, der das aus dem entstehenden Dampf und dem nicht verdampften Anteil der Flüssigfraktion besteht, unterhalb des Sammelbodens, bevorzugt in einem Bereich von 0,15 m bis 0,70 m unterhalb des Sammelbodens, besonders bevorzugt in einem Bereich von 0,20 m bis 0,60 m unterhalb des Sammelbodens, ganz besonders bevorzugt in einem Bereich von 0,25 m bis 0,50 m unterhalb des Sammelbodens, liegt. Dieser Abstand bezieht sich auf den Abstand zweier horizontaler Ebenen, wobei der tiefste Punkt, den die Flüssigfraktion auf dem Sammelboden erreicht, in der oberen Ebene liegt und der tiefste Punkt des Auslasses des Verdampfers in der unteren Ebene liegt. Vorzugsweise bringt man den ersten Wärmetauscher nicht zu weit unten an. Dadurch wird mit dazu beigetragen, dass die Bauhöhe der Extraktivdestillationskolonne nicht vergrößert werden muss. Somit wird ein Optimum hinsichtlich Kosten- und Energieaufwand einerseits und Kolonnenbauhöhe andererseits, erreicht.

Die Sumpffraktion wird nacheinander in einem zweiten indirekten Wärmetauscher und einem dritten indirekten Wärmetauscher erwärmt und teilweise verdampft, wobei der entstehende Dampf mindestens teilweise in die Kolonne freigesetzt wird. Dies kann erfolgen, indem man den Dampf mindestens teilweise in den Sumpf und/oder in den Bereich zwischen dem Sumpf und dem Sammelboden freisetzt.

Die Sumpffraktion kann auf verschiedene Weise nacheinander durch den zweiten Wärmetauscher und den dritten Wärmetauscher geführt werden, wie im Folgenden dargelegt.

Man kann die Sumpffraktion über einen Sumpfumlauf umwälzen, in dem man die Sumpffraktion nacheinander durch den zweiten indirekten Wärmetauscher und den dritten indirekten Wärmetauscher führt. Wenn man die Sumpffraktion in dem einen Sumpfumlauf nacheinander durch den zweiten indirekten Wärmetauscher und den dritten indirekten Wärmetauscher führt, wird der im zweiten Wärmetauscher entstehende Dampf nicht von der Sumpffraktion abgetrennt, bevor die Sumpffraktion in den dritten Wärmetauscher eintritt. In den dritten Wärmetauscher strömt also ein flüssig/dampfförmig-gemischtphasige Strom aus dem zweiten Verdampfer. Im dritten Wärmetauscher wird ein weiterer Anteil der Sumpffraktion verdampft.

Man kann die so erwärmte Sumpffraktion anschließend durch einen Stromteiler führen, um aus dem Sumpfumlauf die dem Stripper zuzuführende Sumpffraktion abzutrennen.

Man kann die Sumpffraktion nach dem dritten indirekten Wärmetauscher durch eine dampfseitig mit der Kolonne kommunizierende Zelle führen, in der man den entstehenden Dampf von der Sumpffraktion abtrennt und aus der man die dem Stripper zuzuführende Sumpffraktion entnimmt. Die Zelle kann in den Sumpf der Kolonne integriert sein oder ein außerhalb der Kolonne angeordneter Phasenabscheider sein.

Wenn die Zelle in den Sumpf der Kolonne integriert ist, wird die dampfseitige Kommunikation der Zelle mit der Kolonne vorzugsweise dadurch erreicht, dass die Zelle zum über dem Sumpf der Kolonne befindlichen Dampf hin geöffnet ist.

Wenn die Zelle ein außerhalb der Kolonne angeordneter Phasenabscheider ist, wird die dampfseitige Kommunikation der Zelle mit der Kolonne vorzugsweise dadurch erreicht, dass man den Phasenabscheider über eine Leitung mit der Kolonne verbindet, über die Dampf aus dem Phasenabscheider in die Kolonne entweichen kann. Vorzugsweise mündet die Leitung oberhalb der Oberfläche der sich im Sumpf sammelnden Sumpffraktion in die Kolonne.

Aus der Zelle, durch die man die umgewälzte Sumpffraktion nach dem dritten indirekten Wärmetauscher führt, wird ein Teil der Sumpffraktion in den Sumpf der Kolonne zurückgeführt. Wenn die Zelle in den Sumpf der Kolonne integriert ist, kann dies dadurch erfolgen, dass man einen Teil der durch die Zelle geführten Sumpffraktion in den (übrigen) Sumpf der Kolonne überlaufen lässt. Wenn die Zelle ein außerhalb der Kolonne angeordneter Phasenabscheider ist, kann man einen Teil der Sumpffraktion aus der Zelle in den Sumpf der Kolonne zurückführen, indem man den Phasenabscheider über eine an einen Überlauf angeschlossene Leitung mit der Kolonne verbindet.

Da eine gleichmäßige Beaufschlagung des dritten Wärmetauschers mit einem flüssig/dampfförmig-gemischtphasigen Strom aus dem zweiten Verdampfer mitunter schwierig sein kann, ist eine Zwischenausgasung des flüssig/dampfförmiggemischtphasigen Stroms bevorzugt. So kann man die Sumpffraktion durch drei dampfseitig mit der Kolonne kommunizierende Zellen führen, indem man die Sumpffraktion in einer ersten Zelle sammelt, die Sumpffraktion aus der ersten Zelle abzieht und durch den zweiten Wärmetauscher in die zweite Zelle führt, die Sumpffraktion aus der zweiten Zelle abzieht und durch den dritten Wärmetauscher in die dritte Zelle führt und die dem Stripper zuzuführende Sumpffraktion aus der dritten Zelle abzieht. Dies hat den Vorteil, dass der im zweiten Wärmetauscher entstehende Dampf nicht in den dritten Wärmetauscher gelangt, sondern aus der dampfseitig mit der Kolonne kommunizierenden Zelle direkt in die Kolonne ausgasen kann. In den dritten Wärmetauscher gelangt dann eine im Wesentlichen einphasige flüssige Sumpffraktion, auf welche sich die Wärme besser übertragen lässt.

Die zweite Zelle und die dritte Zelle können unabhängig voneinander in den Sumpf der Kolonne integriert oder außerhalb der Kolonne angeordnete Phasenabscheider sein. Beide Phasenabscheider können räumlich getrennt voneinander oder in einem Behälter angeordnet sein. Wenn die zweite Zelle und die dritte Zelle außerhalb der Kolonne angeordnete Phasenabscheider sind, bildet die in den Sumpf der Kolonne integrierte erste Zelle den Sumpf der Kolonne.

Bei in den Sumpf der Kolonne integrierten Zellen wird die dampfseitige Kommunikation der Zelle mit der Kolonne vorzugsweise dadurch erreicht, dass die jeweilige Zelle zum über dem Sumpf der Kolonne befindlichen Volumen geöffnet ist.

Bei außerhalb der Kolonne angeordnete Phasenabscheidern, kann die dampfseitige Kommunikation der Zelle mit der Kolonne dadurch erreicht werden, dass man den Phasenabscheider über eine Leitung mit der Kolonne verbindet, über die Dampf aus dem Phasenabscheider in die Kolonne entweichen kann. Alternativ kann die dampfseitige Kommunikation der Zelle mit der Kolonne dadurch erreicht werden, dass man den Phasenabscheider über eine Leitung mit einem anderen Phasenabscheider verbindet, der über eine Leitung mit der Kolonne verbunden ist. Vorzugsweise mündet die jeweilige Leitung oberhalb der Oberfläche der sich im Sumpf sammelnden Sumpffraktion in die Kolonne.

Man kann einen Teil der Sumpffraktion in vorhergehende Zellen zurückführen. Beispielsweise aus der zweiten in die erste Zelle, aus der dritten in die erste Zelle, und/oder aus der dritten in die zweite Zelle.

So kann man z.B. einen Teil der Sumpffraktion aus einem oder gegebenenfalls beiden Phasenabscheidern in die Kolonne führen, beispielsweise durch je einen Überlauf. Der Überlauf kann über eine Leitung in den vorhergehenden Phasenabscheider oder in die Kolonne münden. So kann man einen Teil der Sumpffraktion aus der zweiten in die erste Zelle, aus der dritten in die erste Zelle, und/oder aus der dritten in die zweite Zelle überlaufen lassen.

In einer Ausführungsform des erfindungsgemäßen Verfahrens sind die drei Zellen in den Sumpf der Kolonne integriert.

Unabhängig davon, in welcher Weise die Sumpffraktion nacheinander durch den zweiten Wärmetauscher und den dritten Wärmetauscher geführt wird, kann jede in den Sumpf der Kolonne integrierte Zelle vom übrigen Sumpf der Kolonne bzw. von einer oder mehreren anderen in den Sumpf der Kolonne integrierten Zellen durch jedwede geeignete flüssigkeitsundurchlässige Barriere abgetrennt sein.

Bevorzugte flüssigkeitsundurchlässige Barriere ist eine Trennwand, insbesondere eine vertikale Trennwand. Vorzugsweise besteht die Trennwand aus Metall. Die Trennwand kann insbesondere als konzentrischer Zylinder oder als ebene Trennwand ausgebildet sein, die, im Kolonnenquerschnitt betrachtet, entlang von einem Kreisradius oder einer Kreissehne angeordnet ist.

Vorzugsweise lässt man über die flüssigkeitsundurchlässige Barriere einen Teil der Sumpffraktion aus einer in den Sumpf der Kolonne integrierten Zelle in den übrigen Sumpf der Kolonne oder in eine vorhergehende Zelle überlaufen.

Die B und Extraktionsmittel enthaltende Sumpffraktion wird dann in einem Stripper in eine B enthaltende Fraktion und eine Extraktionsmittelfraktion aufgetrennt. Man beheizt den Stripper, insbesondere den Sumpf des Strippers, um die Auftrennung in die beiden Fraktionen zu erreichen. Man kann den Stripper z.B. über einen Sumpfumlauf beheizen. Den Sumpfumlauf kann man beispielsweise über einen Wärmetauscher beheizen, in dem Wasserdampf als Heizmedium eingesetzt wird. Man kann die Auftrennung in die beiden Fraktionen in dem Stripper durch Strippen und/oder Druckverminderung erleichtern.

Vorzugsweise leitet man die B enthaltende Fraktion am Kopf des Strippers aus. In der B enthaltenden Fraktion ist der Stoffmengenanteil B/(A+B) höher, vorzugsweise mindestens 1,3 mal höher, insbesondere mindestens 1,5 mal höher, als im A und B enthaltenden Zulaufstrom. Den Stoffmengenanteil B/(A+B) berechnet man, indem man die Stoffmenge B durch die Summe der Stoffmenge A und der Stoffmenge B teilt.

Vorzugsweise zieht man eine regenerierte Extraktionsmittelfraktion am Sumpf des Strippers ab.

Die regenerierte Extraktionsmittelfraktion nutzt man als Heizmedium für den zweiten Wärmetauscher, wobei eine teilweise abgekühlte Extraktionsmittelfraktion erhalten wird. Da ein Teil der Wärme aus der Extraktionsmittelfraktion in dem zweiten Wärmetauscher auf die Sumpffraktion übertragen wird, erfolgt eine teilweise Abkühlung der Extraktionsmittelfraktion.

Vorzugsweise dimensioniert man den zweiten Wärmetauscher so, dass die Temperatur der aus dem zweiten Wärmetauscher ausströmenden teilweise abgekühlten Extraktionsmittelfraktion höchstens 30 K, z.B. 1 bis 25 K, insbesondere 2 bis 20 K, vorzugsweise 3 bis 15 K oberhalb der Temperatur der aus dem Wärmetauscher ausströmenden Sumpffraktion liegt.

Für den dritten Wärmetauscher verwendet man ein externes Heizmedium. Mit Hilfe des externen Heizmediums stellt man die gewünschte Sumpftemperatur ein. Die in den dritten Wärmetauscher eintretende Sumpffraktion ist aufgrund des vorhergehenden zweiten Wärmetauschs vorerwärmt. Somit ist die für die Einstellung der gewünschten Sumpftemperatur zusätzlich zuzuführende Wärmemenge auf ein Minimum beschränkt.

Im erfindungsgemäßen Verfahren kann jede zu Übertragung von Wärme von einer Flüssigkeit auf eine andere Flüssigkeit geeignete Form von indirektem Wärmetauscher als zweiter oder dritter Wärmtauscher eingesetzt werden. Beide Wärmetauscher können unabhängig Fallfilmverdampfer, Kesselverdampfer, Zwangsumlaufverdampfer oder Naturumlaufverdampfer sein. Vorzugsweise ist mindestens der zweite oder der dritte Wärmetauscher ein Naturumlaufverdampfer oder ein Fallfilmverdampfer. In Fallfilmverdampfern ist die Verweilzeit der Sumpffraktion besonders kurz, wodurch ein besonders schonendes Aufheizen bzw. Verdampfen gewährleistet werden kann. Deshalb werden Fallfilmverdampfer insbesondere bei hitzeempfindlichen Extraktionsmitteln bevorzugt eingesetzt. Dies gilt insbesondere für den dritten Wärmetauscher, da das für den dritten Wärmetauscher verwendete externe Heizmedium im Allgemeinen heißer ist, als die für den ersten und zweiten Wärmetausch genutzten Heizmedien.

Die teilweise abgekühlte Extraktionsmittelfraktion nutzt man als Heizmedium für den ersten Wärmetauscher. Hierzu führt man die aus dem zweiten Wärmetauscher ausströmende teilweise abgekühlte Extraktionsmittelfraktion durch den ersten Wärmetauscher.

Aus dem ersten Wärmetauscher wird eine abgekühlte Extraktionsmittelfraktion erhalten. Vorzugsweise dimensioniert man den ersten Wärmetauscher so, dass die Temperatur der aus dem ersten Wärmetauscher ausströmenden abgekühlten Extraktionsmittelfraktion höchstens 30 K, z.B. 1 bis 25 K, insbesondere 2 bis 20 K, vorzugsweise 3 bis 15 K über der Temperatur des aus dem Wärmetauscher ausströmenden nicht verdampften Anteils der Flüssigfraktion liegt.

Um den Extraktionsmittelkreislauf zu schließen, recycliert man mindestens einen Teil der aus dem ersten indirekten Wärmetauscher erhaltenen abgekühlten Extraktionsmittelfraktion in den Schritt a). Vor dem Eintritt in die Extraktivdestillationskolonne wird die abgekühlte Extraktionsmittelfraktion vorzugsweise weiter gekühlt.

Bei A und B handelt es sich um verdampf- und kondensierbare bzw. lösliche Stoffe, d.h. um Stoffe, die sich vom flüssigen bzw. gelösten in den gasförmigen Aggregatzustand überführen lassen, und umgekehrt.

Vorzugsweise handelt es sich bei A und B um Stoffe, deren Siedetemperatur sich bei Normaldruck, also bei 1013,25 hPa um z. B. höchstens 25 K, insbesondere höchstens 20 K, vorzugsweise höchstens 15 K unterscheiden, oder um Stoffe, die ein Azeotrop bilden.

A kann eines oder mehrere Isomere umfassen, beispielsweise verschiedene isomere Butane, wenn A für Butan steht.

B kann eines oder mehrere Isomere umfassen, beispielsweise verschiedene isomere Butene, wenn B für Buten, Butadien oder deren Mischungen steht.

Bei dem erfindungsgemäßen Verfahren kann es sich um ein Verfahren handeln, wobei
i. A für Butan und B für Buten, Butadien oder deren Mischungen steht, oder
ii. A für Pentan und B für Penten, Isopren oder deren Mischungen steht, oder
iii. A für Hexan und B für Benzol steht, oder
iv. A für 2-Propanol und B für Wasser steht, oder
v. A für Chlorwasserstoff und B für Wasser steht, oder
vi. A für Acetonitril und B für Wasser steht, oder
vii. A für 2-Butylacetat und B für 2-Butanol, oder
viii. A für Tetrahydrofuran und B für Methanol steht, oder
ix. A für Aceton und B für Chloroform steht, oder
x. A für Ethanol und B für Wasser steht, oder
xi. A für Aceton und B für Methanol steht, oder
xii. A für eine Mischung von Ethanol und Wasser und B für Ethylacetat steht.

In dem erfindungsgemäßen Verfahren hat das Extraktionsmittel eine höhere Affinität zu B als zu A. D.h., dass das Extraktionsmittel mit B stärkere Wechselwirkungen eingeht, als mit A. Die Affinität des Extraktionsmittels zu A bzw. zu B kann der Fachmann z.B. dadurch bestimmen, dass er die Löslichkeit von A bzw. B im Extraktionsmittel bestimmt. Beispielsweise kann er je ein Aliquot Extraktionsmittel unter einer Atmosphäre von A und einer Atmosphäre von B rühren und ermitteln, wie viel von A bzw. B sich im Extraktionsmittel gelöst hat.

Im Allgemeinen ist die Siedetemperatur des Extraktionsmittels bei Normalbedingungen um mindestens 40 K, insbesondere mindestens 50 K, bevorzugt mindestens 60 K, besonders bevorzugt mindestens 80 K höher, als die Siedetemperaturen beider Stoffe A und B bei Normalbedingungen.

Je nach den oben unter i. bis xiii. genannten Stoffen A und B werden teils unterschiedliche Extraktionsmittel bevorzugt, wobei das bevorzugte Extraktionsmittel im Fall von
i. unter Dimethylformamid, N-Methylpyrrolidon, Acetonitril, Furfural und Dimethylsulfoxid ausgewählt ist,
ii. unter Dimethylformamid und N-Methylpyrrolidon ausgewählt ist,
iii. unter Phenol, N-Methylpyrrolidon und N-Formylmorpholin ausgewählt ist
iv. eine Lösung von Calciumchlorid in Wasser ist, oder unter unter Dimethylsulfoxid und Ethylenglycol ausgewählt ist,
v. Schwefelsäure ist,
vi. Ethylenglycol ist,
vii. N,N-Dimethylacetamid ist,
viii. Wasser ist,
ix. Dimethylsulfoxid ist,
x. unter Ethylenglycol, Glycerin und Dimethylsulfoxid ausgewählt ist,
xi. Wasser ist,
xii. unter Dimethylsulfoxid, Glycerin und Diethylenglycol, 1-Naphthol, Hydrochinon und Dimethylformamid ausgewählt ist.

In einer besonders bevorzugten Ausführungsform des erfindungsgemäßen Verfahrens steht A für Butan und B für Buten, Butadien oder deren Mischungen und das Extraktionsmittel umfasst N-Methylpyrrolidon. Vorzugsweise enthält der Zulaufstrom Butan, Buten und/oder Butadien, N-Methylpyrrolidon, und Wasser, und das Extraktionsmittel 80 bis 97 Gew.-% N-Methylpyrrolidon und 3 bis 20 Gew.-% Wasser.

Ein Butan, Buten und Butadien enthaltender Zulaufstrom wird beispielsweise durch katalytische Dehydrierung von n-Butan erhalten. Es kann sich um eine nicht-oxidative, katalytische Dehydrierung oder eine oxidative katalytische Dehydrierung handeln. Die dabei erhaltenen Gemische enthalten in der Regel neben Butan, Buten und Butadien Inertgase wie Wasserstoff, Stickstoff und/oder Kohlendioxid. Eine Abtrennung der Inertgase kann durch Absorption des Buten und/oder Butadien, Butan, Wasserstoff und/oder Stickstoff und gegebenenfalls Kohlendioxid enthaltenden Stoffstroms in einem Absorptionsmittel und anschließende Desorption von Buten, Butadien und Butan erfolgen.

In einer Ausführungsform verwendet man als Absorptionsmittel ein Lösungsmittel bzw. Lösungsmittelgemisch, das als Extraktionsmittel in der anschließenden Extraktivdestillation verwendet wird. Das Absorptionsmittel enthält vorzugsweise 80 bis 97 Gew.-% N-Methylpyrrolidon und 3 bis 20 Gew.-% Wasser. Das mit Buten, Butadien und Butanbeladene Absorptionsmittel wird als Zulaufstrom in die Extraktivdestillation geleitet. Man bildet einen Butan, Buten und/oder Butadien enthaltenden Zulaufstrom, indem man in einer Absorptionskolonne ein Butan, Buten und/oder Butadien, und Wasserstoff und/oder Stickstoff enthaltendes Fluid mit dem flüssigen Absorptionsmittel in Kontakt bringt. Man kann das Fluid in einen unteren Bereich einer Absorptionskolonne und das Absorptionsmittel in einen oberen Bereich der Absorptionskolonne führen, wobei man am Kopf der Absorptionskolonne einen wasserstoffhaltigen Kopfstrom und aus dem Sumpf der Absorptionskolonne den Zulaufstrom erhält.

Man kann das Absorptionsmittel und das Extraktionsmittel durch Aufteilung der aus dem ersten indirekten Wärmetauscher erhaltenen abgekühlten Extraktionsmittelfraktion bilden, beispielsweise indem man die vom ersten indirekten Wärmetauscher abströmende Extraktionsmittelfraktion durch einen Stromteiler führt, in dem die Extraktionsmittelfraktion in einen Extraktionsmittel- und einen Absorptionsmittelstrom aufgeteilt wird. Sowohl vor dem Stromteiler, als auch nach dem Stromteiler kann eine weitere Kühlung erfolgen.

Die Erfindung wird durch die beigefügten Zeichnungen näher veranschaulicht.
Figur 1 zeigt schematisch eine Anlage zur Durchführung eines erfindungsgemäßen Verfahrens.
Figur 2 zeigt schematisch bestimmte Ausführungsformen der in Figur 1 dargestellten Kolonne 3.

Die Figuren 2A-2C zeigen jeweils bevorzugte Anordnungen für die Trennwände zur Aufteilung des Sumpfes der Kolonne der Figur 2.

Die Figuren 3 bis 9 zeigen schematisch weitere Ausführungsformen der in Figur 1 dargestellten Kolonne 3.

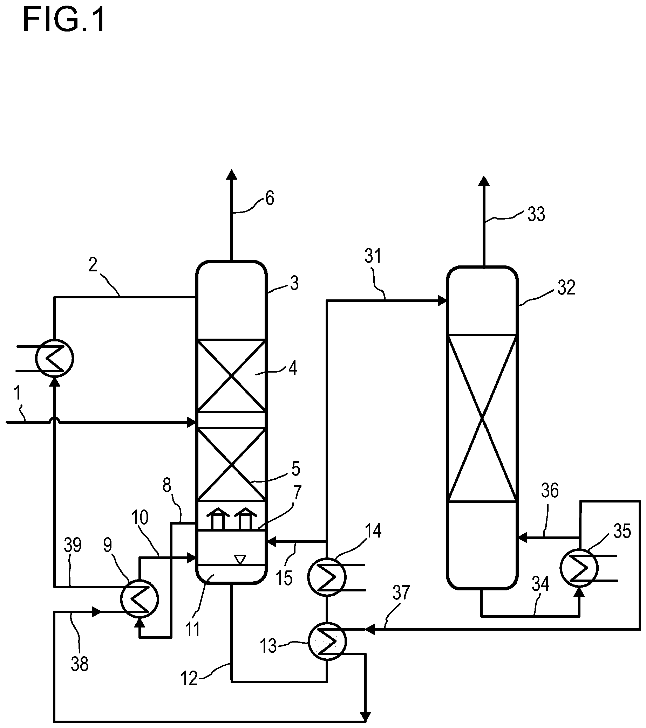

In der in Figur 1 dargestellten Anlage führt man den A und B enthaltenden Zulaufstrom 1 dem durch Leitung 2 zugeführten Extraktionsmittel in der Kolonne 3 entgegen, indem man den Zulaufstrom 1 zwischen der Verstärkungszone 4 und der Abtriebszone 5 in die Kolonne 3 und das Extraktionsmittel 2 oberhalb der Verstärkungszone 4 in die Kolonne 3 führt. Dabei wird die A enthaltende Kopffraktion 6 sowie die B und Extraktionsmittel enthaltende Flüssigfraktion erhalten. Die Flüssigfraktion sammelt man auf dem Sammelboden 7. Man führt die Flüssigfraktion über Leitung 8 in den ersten indirekten Wärmetauscher 9, in dem sie erwärmt und teilweise verdampft wird. Im ersten Wärmetauscher 9 bildet sich ein aus dem entstehenden Dampf und dem nicht verdampften Anteil der Flüssigfraktion bestehendes zweiphasiges Gemisch. Man führt das zweiphasige Gemisch über Leitung 10 in den zwischen dem Sumpf und dem Sammelboden 7 befindlichen Bereich der Kolonne 3 zurück. Nicht verdampfte Flüssigfraktion sammelt sich als Sumpffraktion 11 im Sumpf der Kolonne 3. Die Sumpffraktion 11 wird nacheinander in einem zweiten indirekten Wärmetauscher 13 und einem dritten indirekten Wärmetauscher 14 erwärmt und teilweise verdampft. Hierzu wälzt man die Sumpffraktion 11 über einen Sumpfumlauf um, in dem man die Sumpffraktion über Leitung 12 aus dem Sumpf der Kolonne abzieht, sie nacheinander durch den zweiten indirekten Wärmetauscher 13 und den dritten indirekten Wärmetauscher 14 und über Leitung 15 führt. Über Leitung 31 führt man einen Teil der umgewälzten Sumpffraktion in den Stripper 32 und trennt sie darin in eine am Kopf des Strippers ausgeleitete B enthaltende Fraktion 33 und eine Extraktionsmittelfraktion auf. Den Sumpf des Strippers beheizt man über einen Sumpfumlauf (34, 35, 36), über den man die Extraktionsmittelfraktion umwälzt und in dem man sie über Wärmetauscher 35 erwärmt. Man nutzt die Extraktionsmittelfraktion als Heizmedium für den zweiten Wärmetauscher, indem man sie über Leitung 37 und durch den zweiten Wärmetauscher 13 führt. Dabei erhält man eine teilweise abgekühlte Extraktionsmittelfraktion. Für den dritten Wärmetauscher 14 verwendet man ein externes Heizmedium. Man nutzt die teilweise abgekühlte Extraktionsmittelfraktion als Heizmedium für den ersten Wärmetauscher, indem man sie über Leitung 38 und durch den ersten Wärmetauscher 9 führt. Man recycliert die aus dem ersten Wärmetauscher 9 erhaltenen abgekühlten Extraktionsmittelfraktion nach weiterer Kühlung, indem man die aus dem ersten Wärmetauscher austretende abgekühlte Extraktionsmittelfraktion durch Leitung 39 in Kühler 40 führt, um das Extraktionsmittel zu bilden, das über Leitung 2 zugeführt wird.

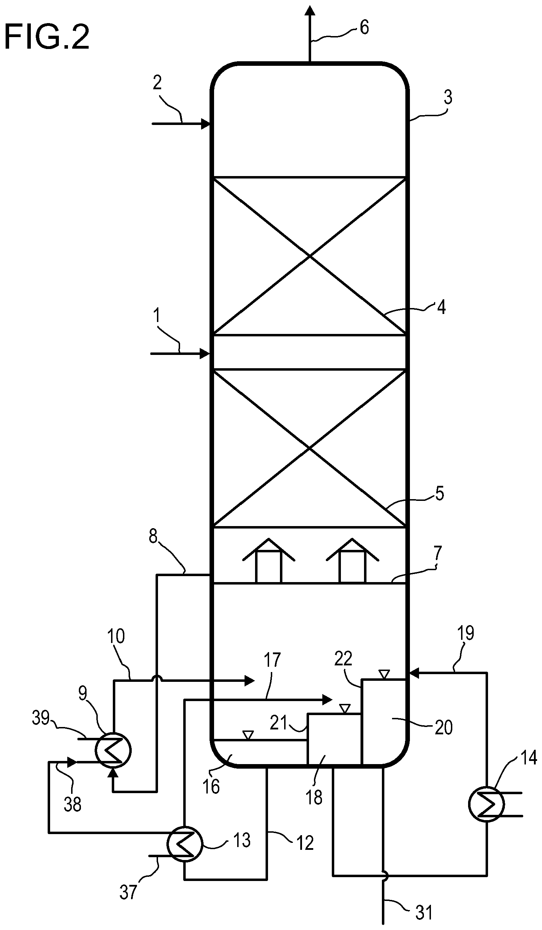

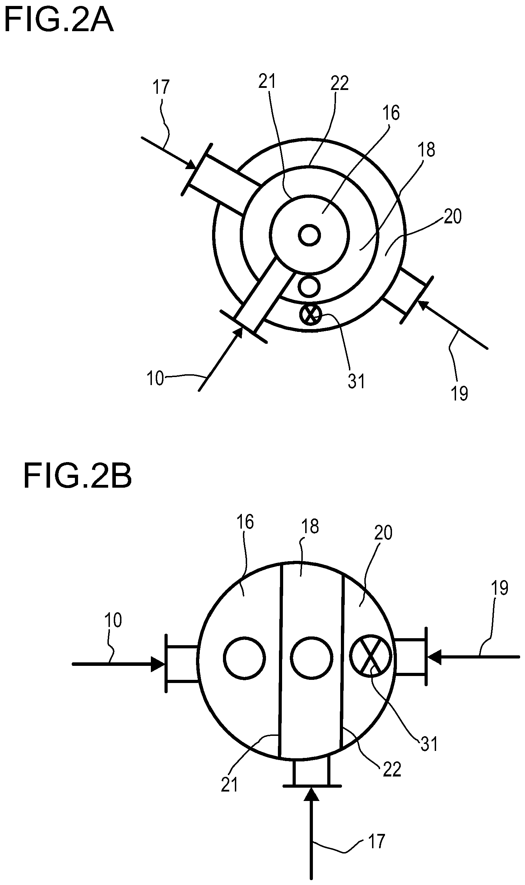

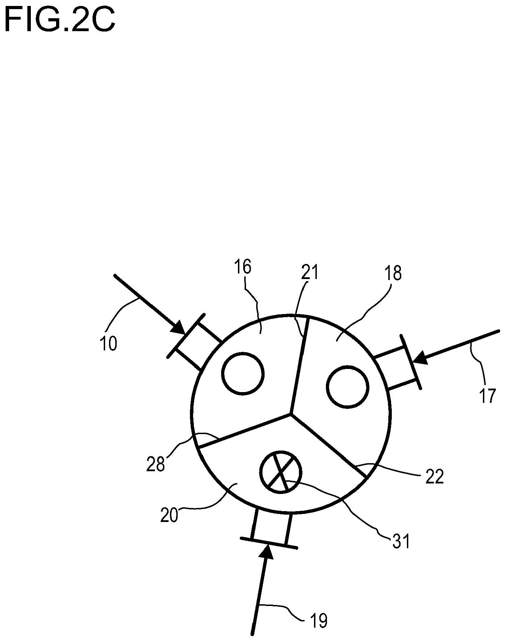

Die in Figuren 2 bis 7 dargestellten Ausführungsformen der Kolonne 3 ermöglichen eine Verfahrensführung, wobei man die Sumpffraktion durch drei dampfseitig mit der Kolonne kommunizierende Zellen führt, indem man die Sumpffraktion in einer in den Sumpf der Kolonne integrierten ersten Zelle sammelt, die Sumpffraktion aus der ersten Zelle abzieht und durch den zweiten Wärmetauscher in die zweite Zelle führt, die Sumpffraktion aus der zweiten Zelle abzieht und durch den dritten Wärmetauscher in die dritte Zelle führt und die dem Stripper zuzuführende Sumpffraktion aus der dritten Zelle abzieht.

In der in Figur 2 dargestellten Ausführungsform der Kolonne 3 sind alle drei Zellen in den Sumpf der Kolonne integriert. Leitung 10 mündet oberhalb der ersten Zelle 16. Man führt das im ersten Wärmetauscher 9 aus der Flüssigfraktion gebildete zweiphasiges Gemisch über Leitung 10 in den zwischen dem Sumpf und dem Sammelboden 7 befindlichen Bereich oberhalb der ersten Zelle 16, so dass sich der nicht verdampfte Anteil der Flüssigfraktion als Sumpffraktion in der ersten Zelle 16 sammelt und der entstehende Dampf in die Kolonne 3 freigesetzt wird. Die Sumpffraktion zieht man über Leitung 12 aus der ersten Zelle 16 ab und führt sie durch den zweiten Wärmetauscher 13 und über Leitung 17 in den zwischen dem Sumpf und dem Sammelboden befindlichen Bereich oberhalb der zweiten Zelle 18, so dass sich der nicht verdampfte Anteil der Sumpffraktion in der zweiten Zelle 18 sammelt und der entstehende Dampf in die Kolonne 3 freigesetzt wird. Die erste Zelle 16 ist von der zweiten Zelle 18 durch eine Trennwand 21 abgetrennt. Aus der zweiten Zelle 18 zieht man die Sumpffraktion ab und führt sie durch den dritten Wärmetauscher 14 und über Leitung 19 in den zwischen dem Sumpf und dem Sammelboden befindlichen Bereich oberhalb der dritten Zelle 20, so dass sich der nicht verdampfte Anteil der Sumpffraktion in der dritten Zelle 20 sammelt und der entstehende Dampf in die Kolonne 3 freigesetzt wird. Die zweite Zelle 18 ist von der dritten Zelle 20 durch eine Trennwand 22 abgetrennt. Die dem Stripper über Leitung 31 zuzuführende Sumpffraktion zieht man aus der dritten Zelle ab. Wenn der dritten Zelle 20 über Leitung 19 mehr Sumpffraktion zugeführt wird, als über Leitung 31 abgezogen wird, kann die Sumpffraktion aus der dritten Zelle 20 über die Trennwand 22 in die zweite Zelle 18 überlaufen. Wenn der zweiten Zelle 18 über Leitung 17 mehr Sumpffraktion zugeführt wird, als aus der zweiten Zelle 18 abgezogen wird, kann die Sumpffraktion aus der zweiten Zelle 18 über die Trennwand 20 in die erste Zelle 16 überlaufen.

Die Querschnittsdarstellungen in den Figuren 2A-2C zeigen jeweils bevorzugte Anordnungen für die Trennwände zur Aufteilung des Kolonnensumpfes in die drei Zellen 16, 18 und 20: eine konzentrische Anordnung der Trennwände in Figur 2A, eine Anordnung in Form von Kreissehnen in Figur 2B bzw. in Form von Kreisradien in Figur 2C.

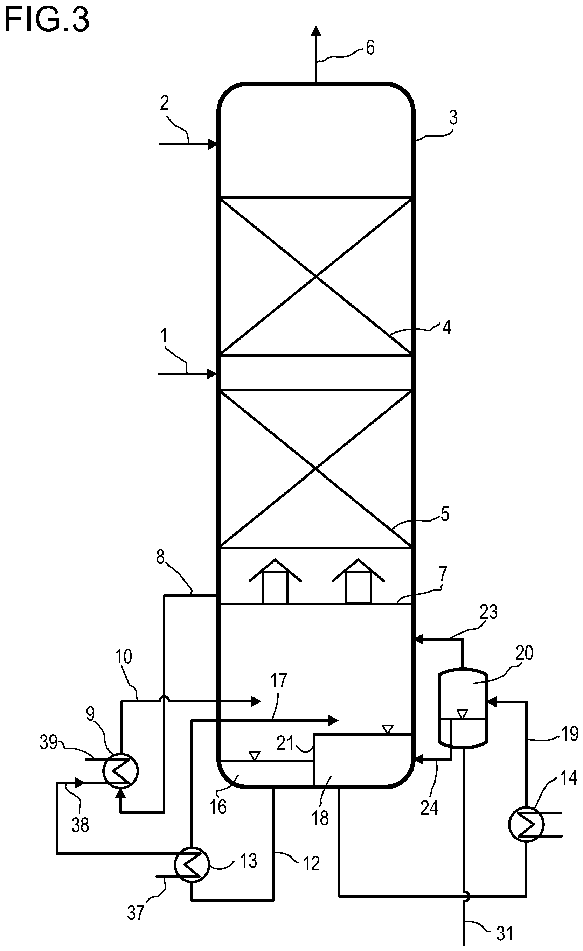

In der in Figur 3 dargestellten Ausführungsform der Kolonne 3 sind die erste Zelle 16 und die zweite Zelle 18 in den Sumpf der Kolonne integriert. Die dritte Zelle 20 ist ein außerhalb der Kolonne angeordneter Phasenabscheider. Leitung 10 mündet oberhalb der ersten Zelle 16. Man führt das im ersten Wärmetauscher 9 aus der Flüssigfraktion gebildete zweiphasiges Gemisch über Leitung 10 in den zwischen dem Sumpf und dem Sammelboden 7 befindlichen Bereich oberhalb der ersten Zelle 16, so dass sich der nicht verdampfte Anteil der Flüssigfraktion als Sumpffraktion in der ersten Zelle 16 sammelt und der entstehende Dampf in die Kolonne 3 freigesetzt wird. Die Sumpffraktion zieht man über Leitung 12 aus der ersten Zelle 16 ab und führt sie durch den zweiten Wärmetauscher 13 und über Leitung 17 in den zwischen dem Sumpf und dem Sammelboden befindlichen Bereich oberhalb der zweiten Zelle 18, so dass sich der nicht verdampfte Anteil der Sumpffraktion in der zweiten Zelle 18 sammelt und der entstehende Dampf in die Kolonne 3 freigesetzt wird. Die erste Zelle 16 ist von der zweiten Zelle 18 durch eine Trennwand 21 abgetrennt. Aus der zweiten Zelle 18 zieht man die Sumpffraktion ab und führt sie durch den dritten Wärmetauscher 14 und über Leitung 19 in den Phasenabscheider. Der Phasenabscheider weist einen Überlauf auf, der in Leitung 24 mündet. Durch den Überlauf lässt man Sumpffraktion über Leitung 24 aus dem Phasenabscheider in die zweite Zelle 18 überlaufen. Der in den Phasenabscheider eingebrachte Überlauf, der über Leitung 24 getaucht in die Kolonne zugeführt wird, wirkt hierbei wie ein Syphon und verhindert das Leerlaufen des Phasenabscheiders solange Leitung 19 mehr Flüssigkeit führt, als Leitung 31. Den sich im Phasenabscheider sammelnden Dampf setzt man über Leitung 23 in die Kolonne frei. Die dem Stripper über Leitung 31 zuzuführende Sumpffraktion zieht man aus dem Phasenabscheider ab.

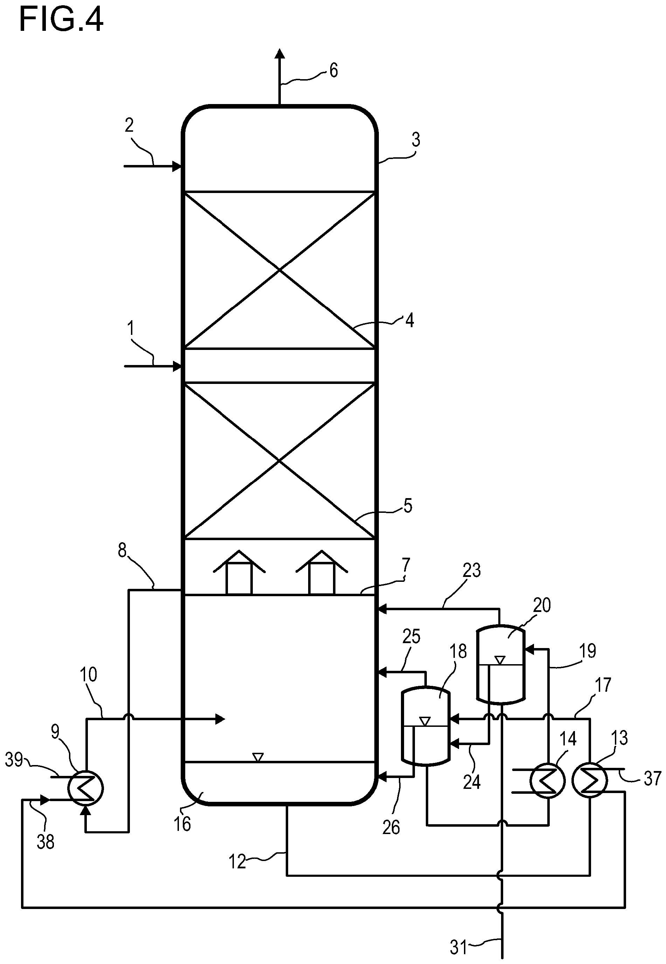

In der in Figur 4 dargestellten Ausführungsform der Kolonne 3 sind die zweite Zelle 18 und die dritte Zelle 20 je außerhalb der Kolonne angeordnete Phasenabscheider. Man sammelt den nicht verdampften Anteil der Flüssigfraktion als Sumpffraktion in der in den Sumpf der Kolonne integrierten ersten Zelle 16. Aus der ersten Zelle 16 zieht man die Sumpffraktion über Leitung 12 ab und führt sie durch den zweiten Wärmetauscher 13 und über Leitung 17 in den Phasenabscheider 18. Aus diesem zieht man die Sumpffraktion ab und führt sie durch den dritten Wärmetauscher 14 und über Leitung 19 in den Phasenabscheider 20. Die Phasenabscheider weisen je einen Überlauf auf, durch die man Sumpffraktion über Leitung 26 aus dem Phasenabscheider 18 in die erste Zelle 16 sowie über Leitung 24 aus der dem Phasenabscheider 20 in den Phasenabscheider 18 überlaufen lässt. Die sich in den Phasenabscheidern sammelnden Dämpfe setzt man über Leitungen 23 und 25 in die Kolonne frei. Die dem Stripper über Leitung 31 zuzuführende Sumpffraktion zieht man aus dem Phasenabscheider 20 ab.

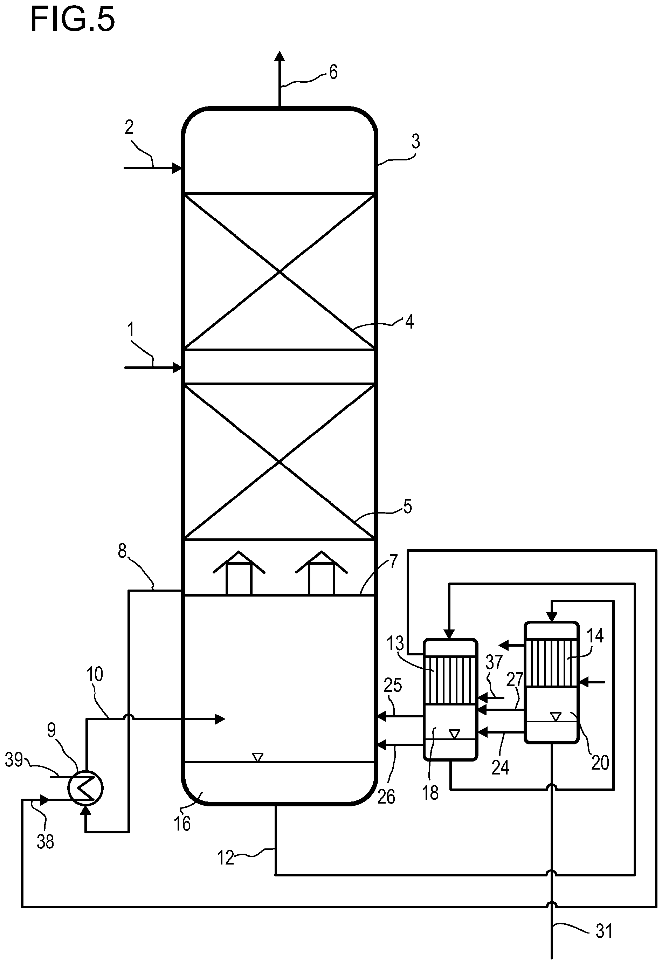

In der in Figur 5 dargestellten Ausführungsform der Kolonne 3 sind die zweite Zelle 18 und die dritte Zelle 20 je außerhalb der Kolonne angeordnete Phasenabscheider. Der zweite und dritte Wärmetauscher 13 und 14 sind in den oberen Bereich der Phasenabscheider 18 und 20 integriert. Aus der ersten Zelle 16 zieht man die Sumpffraktion über Leitung 12 ab, führt sie in den Phasenabscheider 18 und darin durch den zweiten Wärmetauscher 13. Aus der zweiten Zelle 18 zieht man die Sumpffraktion ab, führt sie in den Phasenabscheider 20 und darin durch den dritten Wärmetauscher 14. Beide Wärmetauscher sind Fallfilmverdampfer, die von der Sumpffraktion von oben nach unten durchströmt werden. Über Leitung 25 führt man Sumpffraktion aus dem Phasenabscheider 20 in den Phasenabscheider 18. Über Leitung 26 führt man Sumpffraktion aus dem Phasenabscheider 18 in die erste Zelle 16. Den sich im Phasenabscheider 18 sammelnden Dampf setzt man über Leitung 25 in die Kolonne frei. Den sich im Phasenabscheider 20 sammelnden Dampf führt man über Leitung 25 in den Phasenabscheider 18. Die dem Stripper über Leitung 31 zuzuführende Sumpffraktion zieht man aus dem Phasenabscheider 20 ab.

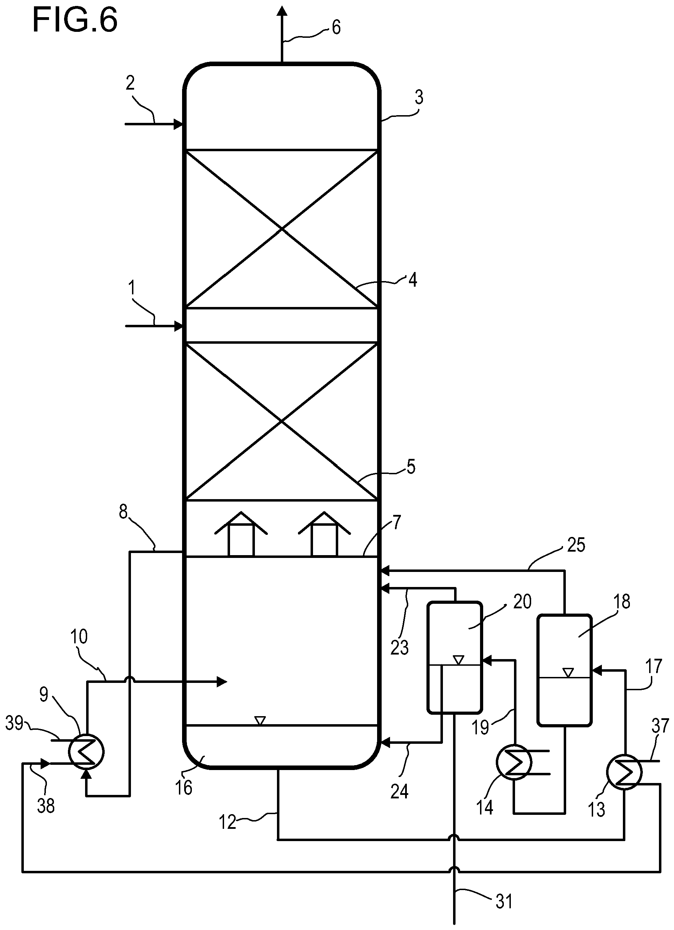

In der in Figur 6 dargestellten Ausführungsform der Kolonne 3 sind die zweite Zelle 18 und die dritte Zelle 20 ja außerhalb der Kolonne angeordnete Phasenabscheider. Man sammelt den nicht verdampften Anteil der Flüssigfraktion als Sumpffraktion in der in den Sumpf der Kolonne integrierten ersten Zelle 16. Aus der ersten Zelle 16 zieht man die Sumpffraktion über Leitung 12 ab und führt sie durch den zweiten Wärmetauscher 13 und über Leitung 17 in den Phasenabscheider 18. Aus der zweiten Zelle 18 zieht man die Sumpffraktion ab und führt sie durch den dritten Wärmetauscher 14 und über Leitung 19 in den Phasenabscheider 20. Der Phasenabscheider 20 weist einen Überlauf auf, durch die man Sumpffraktion über Leitung 24 in die erste Zelle 16 überlaufen lässt. Die sich in den Phasenabscheidern sammelnden Dämpfe setzt man über Leitungen 23 und 25 in die Kolonne frei. Die dem Stripper über Leitung 31 zuzuführende Sumpffraktion zieht man aus Phasenabscheider 20 ab.

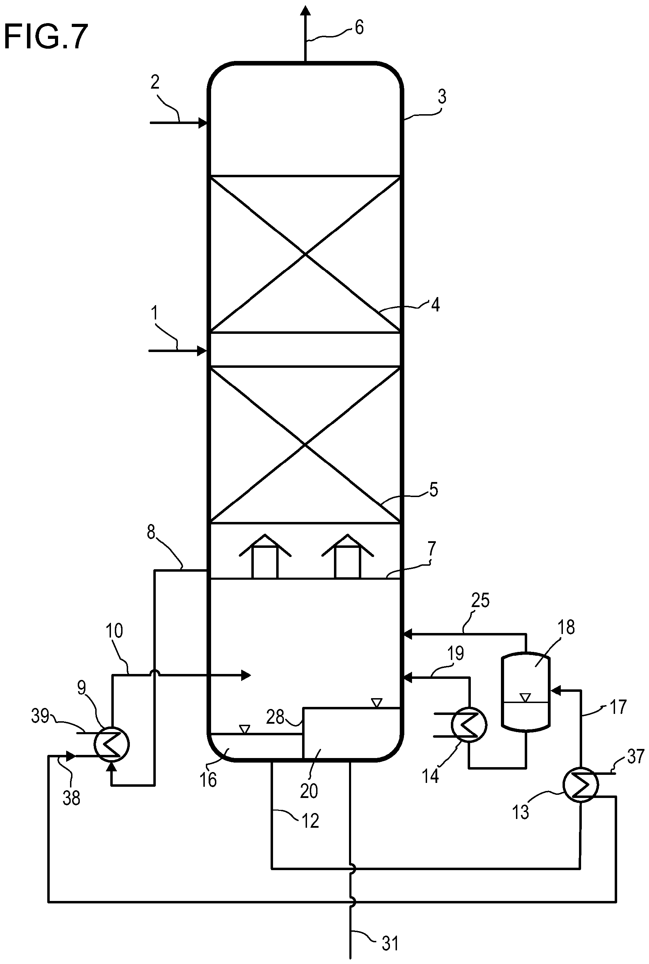

In der in Figur 7 dargestellten Ausführungsform der Kolonne 3 sind die erste Zelle 16 und die dritte Zelle 20 in den Sumpf der Kolonne integriert. Die zweite Zelle 18 ist ein außerhalb der Kolonne angeordneter Phasenabscheider. Man führt das im ersten Wärmetauscher 9 aus der Flüssigfraktion gebildete zweiphasiges Gemisch über Leitung 10 in den zwischen dem Sumpf und dem Sammelboden 7 befindlichen Bereich oberhalb der ersten Zelle 16, so dass sich der nicht verdampfte Anteil der Flüssigfraktion als Sumpffraktion in der ersten Zelle 16 sammelt und der entstehende Dampf in die Kolonne 3 freigesetzt wird. Die Sumpffraktion zieht man über Leitung 12 aus der ersten Zelle 16 ab und führt sie durch den zweiten Wärmetauscher 13 und über Leitung 17 in den Phasenabscheider 18. Aus dem Phasenabscheider 18 zieht man die Sumpffraktion ab und führt sie durch den dritten Wärmetauscher 14 und über Leitung 19 in den zwischen dem Sumpf und dem Sammelboden 7 befindlichen Bereich oberhalb der dritten Zelle 20, so dass sich der nicht verdampfte Anteil der Sumpffraktion in der dritten Zelle 20 sammelt. Den sich im Phasenabscheider sammelnden Dampf setzt man über Leitung 25 in die Kolonne frei. Die dem Stripper über Leitung 31 zuzuführende Sumpffraktion zieht man aus der dritten Zelle 20 ab.

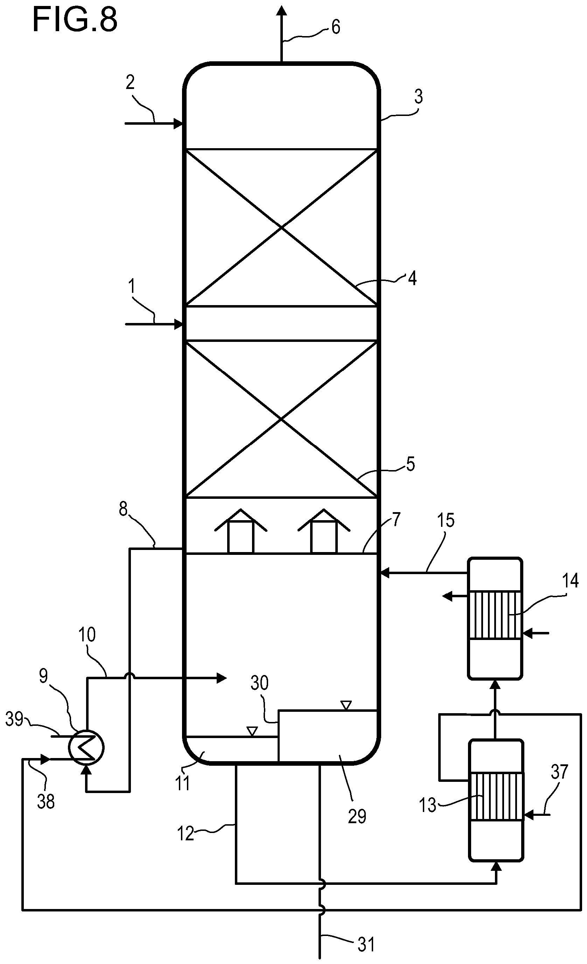

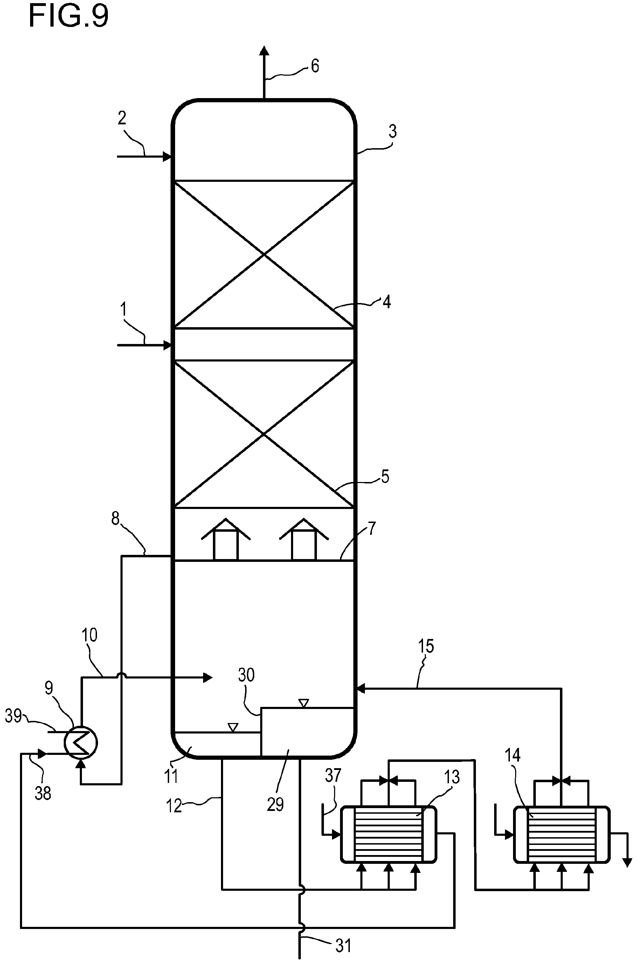

Die in Figuren 8 und 9 dargestellten Ausführungsformen der Kolonne 3 ermöglichen eine Verfahrensführung, bei der man die Sumpffraktion über einen Sumpfumlauf umwälzt, in dem man die Sumpffraktion nacheinander durch den zweiten indirekten Wärmetauscher und den dritten indirekten Wärmetauscher führt und die umgewälzte Sumpffraktion nach dem dritten indirekten Wärmetauscher durch eine in den Sumpf der Kolonne integrierte und somit dampfseitig mit der Kolonne kommunizierende Zelle führt.

In der in Figur 8 dargestellten Ausführungsform der Kolonne 3 führt man das zweiphasige Gemisch über Leitung 10 in den zwischen dem Sumpf und dem Sammelboden 7 befindlichen Bereich der Kolonne 3, so dass sich der nicht verdampfte Anteil der Flüssigfraktion als Sumpffraktion 11 sammelt und der entstehende Dampf in die Kolonne 3 freigesetzt wird. Man wälzt die Sumpffraktion 11 über einen Sumpfumlauf um, in dem man die Sumpffraktion über Leitung 12 aus dem Sumpf der Kolonne abzieht, sie nacheinander durch den zweiten indirekten Wärmetauscher 13 und den dritten indirekten Wärmetauscher 14 und über Leitung 15 in den zwischen dem Sumpf und dem Sammelboden 7 befindlichen Bereich oberhalb der Zelle 29 führt, so dass sich der nicht verdampfte Anteil der Sumpffraktion in der Zelle 29 sammelt. Die dem Stripper über Leitung 31 zuzuführende Sumpffraktion zieht man aus der Zelle 29 ab. Die beiden Wärmetauscher 13 und 14 sind übereinander angeordnete vertikal ausgerichtete Rohrbündelwärmetauscher.

Die in der Figur 9 dargestellte Ausführungsform der Kolonne unterscheidet sich von der in Figur 8 dargestellten Ausführungsform darin, dass die beiden Wärmetauscher 13 und 14 nebeneinander angeordnete horizontal ausgerichtete Rohrbündelwärmetauscher sind.

## Patentansprüche

1. Verfahren zur Trennung eines Gemisches von Stoffen A und B durch Extraktivdestillation unter Verwendung eines Extraktionsmittels mit einer höheren Affinität zu B als zu A, wobei man
a) einen A und B enthaltenden Zulaufstrom dem Extraktionsmittel in einer Kolonne entgegenführt, wobei eine A enthaltende Kopffraktion sowie eine B und Extraktionsmittel enthaltende Flüssigfraktion erhalten wird,
b) die Flüssigfraktion auf einem Sammelboden sammelt und in einem ersten indirekten Wärmetauscher erwärmt und teilweise verdampft, den entstehenden Dampf in die Kolonne freisetzt und einen nicht verdampften Anteil der Flüssigfraktion als Sumpffraktion im Sumpf der Kolonne sammelt,
c) die Sumpffraktion nacheinander in einem zweiten indirekten Wärmetauscher und einem dritten indirekten Wärmetauscher erwärmt und teilweise verdampft, wobei der entstehende Dampf mindestens teilweise in die Kolonne freigesetzt wird,
d) die Sumpffraktion in einem Stripper in eine B enthaltende Fraktion und eine Extraktionsmittelfraktion auftrennt,
e) die Extraktionsmittelfraktion als Heizmedium für den zweiten Wärmetauscher nutzt, wobei eine teilweise abgekühlte Extraktionsmittelfraktion erhalten wird, und ein externes Heizmedium für den dritten Wärmetauscher verwendet, und
f) die teilweise abgekühlte Extraktionsmittelfraktion als Heizmedium für den ersten Wärmetauscher nutzt.

2. Verfahren nach Anspruch 1, wobei man die Sumpffraktion über einen Sumpfumlauf umwälzt, in dem man die Sumpffraktion nacheinander durch den zweiten indirekten Wärmetauscher und den dritten indirekten Wärmetauscher führt.

3. Verfahren nach Anspruch 2, wobei man die umgewälzte Sumpffraktion nach dem dritten indirekten Wärmetauscher durch eine dampfseitig mit der Kolonne kommunizierende Zelle führt, in der man den entstehenden Dampf von der Sumpffraktion abtrennt und aus der man die dem Stripper zugeführte Sumpffraktion entnimmt.

4. Verfahren nach Anspruch 3, wobei die Zelle in den Sumpf der Kolonne integriert oder ein außerhalb der Kolonne angeordneter Phasenabscheider ist.

5. Verfahren nach Anspruch 1, wobei man die Sumpffraktion durch drei dampfseitig mit der Kolonne kommunizierende Zellen führt, indem man die Sumpffraktion in einer in den Sumpf der Kolonne integrierten ersten Zelle sammelt, die Sumpffraktion aus der ersten Zelle abzieht und durch den zweiten Wärmetauscher in die zweite Zelle führt, die Sumpffraktion aus der zweiten Zelle abzieht und durch den dritten Wärmetauscher in die dritte Zelle führt und die dem Stripper zuzuführende Sumpffraktion aus der dritten Zelle abzieht.

6. Verfahren nach Anspruch 5, wobei die drei Zellen in den Sumpf der Kolonne integriert sind.

7. Verfahren nach Anspruch 5, wobei die dritte Zelle ein außerhalb der Kolonne angeordneter Phasenabscheider ist.

8. Verfahren nach Anspruch 5 oder 7, wobei die zweite Zelle ein außerhalb der Kolonne angeordneter Phasenabscheider ist.

9. Verfahren nach einem der vorhergehenden Ansprüche, wobei der erste Wärmetauscher ein Naturumlaufverdampfer ist.

10. Verfahren nach einem der vorhergehenden Ansprüche, wobei
i. A für Butan und B für Buten, Butadien oder deren Mischungen steht, oder
ii. A für Pentan und B für Penten, Isopren oder deren Mischungen steht, oder
iii. A für Hexan und B für Benzol steht, oder
iv. A für 2-Propanol und B für Wasser steht, oder
v. A für Chlorwasserstoff und B für Wasser steht, oder
vi. A für Acetonitril und B für Wasser steht, oder
vii. A für 2-Butylacetat und B für 2-Butanol, oder
viii. A für Tetrahydrofuran und B für Methanol steht, oder
ix. A für Aceton und B für Chloroform steht, oder
x. A für Ethanol und B für Wasser steht, oder
xi. A für Aceton und B für Methanol steht, oder
xii. A für eine Mischung von Ethanol und Wasser und B für Ethylacetat steht.

11. Verfahren nach Anspruch 10, wobei das Extraktionsmittel im Fall von
i. unter Dimethylformamid, N-Methylpyrrolidon, Acetonitril, Furfural und Dimethylsulfoxid ausgewählt ist,
ii. unter Dimethylformamid und N-Methylpyrrolidon ausgewählt ist,
iii. unter Phenol, N-Methylpyrrolidon und N-Formylmorpholin ausgewählt ist
iv. eine Lösung von Calciumchlorid in Wasser ist, oder unter unter Dimethylsulfoxid und Ethylenglycol ausgewählt ist,
v. Schwefelsäure ist,
vi. Ethylenglycol ist,
vii. N,N-Dimethylacetamid ist,
viii. Wasser ist,
ix. Dimethylsulfoxid ist,
x. unter Ethylenglycol, Glycerin und Dimethylsulfoxid ausgewählt ist,
xi. Wasser ist,
xii. unter Dimethylsulfoxid, Glycerin und Diethylenglycol, 1-Naphthol, Hydrochinon und Dimethylformamid ausgewählt ist.

12. Verfahren nach einem der vorhergehenden Ansprüche, wobei A für Butan und B für Buten, Butadien oder deren Mischungen steht und das Extraktionsmittel N-Methylpyrrolidon umfasst.

## Claims

1. A method for separating a mixture of materials A and B by extractive distillation, using an extraction medium having a higher affinity to B than to A, wherein
a) a feed stream comprising A and B is conducted towards the extraction medium in a column, wherein an overhead fraction comprising A and also a liquid fraction comprising B and extraction medium are obtained,
b) the liquid fraction is collected on a collecting tray and heated and partially evaporated in a first indirect heat exchanger, the resultant vapor is released into the column and a non-evaporated proportion of the liquid fraction is collected as sump fraction in the sump of the column,
c) the sump fraction is successively heated in a second indirect heat exchanger and a third indirect heat exchanger and in part evaporated, wherein the resultant vapor is at least in part released into the column,
d) the sump fraction is separated in a stripper into a fraction comprising B and an extraction medium fraction,
e) the extraction medium fraction is used as heating medium for the second heat exchanger, wherein a partially cooled extraction medium fraction is obtained, and an external heating medium is used for the third heat exchanger, and
f) the partially cooled extraction medium fraction is used as heating medium for the first heat exchanger.

2. The method according to claim 1, wherein the sump fraction is circulated via a sump circulation, in which the sump fraction is successively conducted through the second indirect heat exchanger and the third indirect heat exchanger.

3. The method according to claim 2, wherein the circulated sump fraction, downstream of the third indirect heat exchanger, is conducted through a cell communicating on the vapor side with the column, in which cell the resultant vapor is separated off from the sump fraction and from which the sump fraction that is fed to the stripper is withdrawn.

4. The method according to claim 3, wherein the cell is integrated into the sump of the column, or a phase separator is arranged outside the column.

5. The method according to claim 1, wherein the sump fraction is conducted through three cells communicating on the vapor side with the column, by collecting the sump fraction in a first cell integrated into the sump of the column, taking off the sump fraction from the first cell and conducting it through the second heat exchanger into the second cell, taking off the sump fraction from the second cell and conducting it through the third heat exchanger into the third cell and taking off from the third cell the sump fraction that is to be fed to the stripper.

6. The method according to claim 5, wherein the three cells are integrated into the sump of the column.

7. The method according to claim 5, wherein the third cell is a phase separator arranged outside the column.

8. The method according to claim 5 or 7, wherein the second cell is a phase separator arranged outside the column.

9. The method according to any one of the preceding claims, wherein the first heat exchanger is a natural-circulation evaporator.

10. The method according to any one of the preceding claims, wherein
i. A is butane and B is butene, butadiene or mixtures thereof, or
ii. A is pentane and B is pentene, isoprene or mixtures thereof, or
iii. A is hexane and B is benzene, or
iv. A is 2-propanol and B is water, or
v. A is hydrogen chloride and B is water, or
vi. A is acetonitrile and B is water, or
vii. A is 2-butyl acetate and B is 2-butanol, or
viii. A is tetrahydrofuran and B is methanol, or
ix. A is acetone and B is chloroform, or
x. A is ethanol and B is water, or
xi. A is acetone and B is methanol, or
xii. A is a mixture of ethanol and water and B is ethyl acetate.

11. The method according to claim 10, wherein the extraction medium in the case of
i. is selected from dimethylformamide, N-methylpyrrolidone, acetonitrile, furfural and dimethyl sulfoxide,
ii. is selected from dimethylformamide and N-methylpyrrolidone,
iii. is selected from phenol, N-methylpyrrolidone and N-formylmorpholine,
iv. is a solution of calcium chloride in water, or is selected from dimethyl sulfoxide and ethylene glycol,
v. is sulfuric acid,
vi. is ethylene glycol,
vii. is N,N-dimethylacetamide,
viii. is water,
ix. is dimethyl sulfoxide,
x. is selected from ethylene glycol, glycerol and dimethyl sulfoxide,
xi. is water,
xii. is selected from dimethyl sulfoxide, glycerol and diethylene glycol, 1-naphthol, hydroquinone and dimethylformamide.

12. The method according to any one of the preceding claims, wherein A is butane and B is butene, butadiene or mixtures thereof, and the extraction medium comprises N-methylpyrrolidone.

## Revendications

1. Procédé de séparation d'un mélange de matières A et B par distillation extractive en utilisant un agent d'extraction présentant une affinité plus élevée pour B que pour A, dans lequel
a) un courant d'entrée contenant A et B est mis en circulation à contre-courant de l'agent d'extraction dans une colonne, une fraction de tête contenant A et une fraction liquide contenant B et l'agent d'extraction étant obtenues,
b) la fraction liquide est collectée sur un plateau de collecte et chauffée dans un premier échangeur de chaleur indirect et partiellement évaporée, la vapeur formée est libérée dans la colonne et une fraction non évaporée de la fraction liquide est collectée en tant que fraction de fond dans le fond de la colonne,
c) la fraction de fond est chauffée successivement dans un deuxième échangeur de chaleur indirect et un troisième échangeur de chaleur indirect, et partiellement évaporée, la vapeur formée étant au moins partiellement libérée dans la colonne,
d) la fraction de fond est séparée dans un extracteur en une fraction contenant B et une fraction d'agent d'extraction,
e) la fraction d'agent d'extraction est utilisée en tant que milieu chauffant pour le deuxième échangeur de chaleur, une fraction d'agent d'extraction partiellement refroidie étant obtenue, et un milieu chauffant externe est utilisé pour le troisième échangeur de chaleur, et
f) la fraction d'agent d'extraction partiellement refroidie est utilisée en tant que milieu chauffant pour le premier échangeur de chaleur.

2. Procédé selon la revendication 1, dans lequel la fraction de fond est mise en circulation dans un circuit de fond, dans lequel la fraction de fond est acheminée successivement au travers du deuxième échangeur de chaleur indirect et du troisième échangeur de chaleur indirect.

3. Procédé selon la revendication 2, dans lequel la fraction de fond mise en circulation est acheminée après le troisième échangeur de chaleur indirect au travers d'une cellule communiquant avec la colonne du côté de la vapeur, dans laquelle la vapeur formée est séparée de la fraction de fond et à partir de laquelle la fraction de fond introduite dans l'extracteur est soutirée.

4. Procédé selon la revendication 3, dans lequel la cellule est intégrée dans le fond de la colonne ou est un séparateur de phases agencé à l'extérieur de la colonne.

5. Procédé selon la revendication 1, dans lequel la fraction de fond est acheminée au travers de trois cellules communiquant avec la colonne du côté de la vapeur par collecte de la fraction de fond dans une première cellule intégrée dans le fond de la colonne, soutirage de la fraction de fond de la première cellule et acheminement au travers du deuxième échangeur de chaleur dans la deuxième cellule, soutirage de la fraction de fond de la deuxième cellule et acheminement au travers du troisième échangeur de chaleur dans la troisième cellule, et soutirage de la fraction de fond à introduire dans l'extracteur à partir de la troisième cellule.

6. Procédé selon la revendication 5, dans lequel les trois cellules sont intégrées dans le fond de la colonne.

7. Procédé selon la revendication 5, dans lequel la troisième cellule est un séparateur de phases agencé à l'extérieur de la colonne.

8. Procédé selon la revendication 5 ou 7, dans lequel la deuxième cellule est un séparateur de phases agencé à l'extérieur de la colonne.

9. Procédé selon l'une quelconque des revendications précédentes, dans lequel le premier échangeur de chaleur est un évaporateur à circulation naturelle.

10. Procédé selon l'une quelconque des revendications précédentes, dans lequel
i. A désigne le butane et B désigne le butène, le butadiène ou leurs mélanges, ou
ii. A désigne le pentane et B désigne le pentène, l'isoprène ou leurs mélanges, ou
iii. A désigne l'hexane et B désigne le benzène, ou
iv. A désigne le 2-propanol et B désigne l'eau, ou
v. A désigne le chlorure d'hydrogène et B désigne l'eau, ou
vi. A désigne l'acétonitrile et B désigne l'eau, ou
vii. A désigne l'acétate de 2-butyle et B désigne le 2-butanol, ou
viii. A désigne le tétrahydrofurane et B désigne le méthanol, ou
ix. A désigne l'acétone et B désigne le chloroforme, ou
x. A désigne l'éthanol et B désigne l'eau, ou
xi. A désigne l'acétone et B désigne le méthanol, ou
xii. A désigne un mélange d'éthanol et d'eau et B désigne l'acétate d'éthyle.

11. Procédé selon la revendication 10, dans lequel l'agent d'extraction dans le cas
i. est choisi parmi le diméthylformamide, la N-méthylpyrrolidone, l'acétonitrile, le furfural et le diméthylsulfoxyde,
ii. est choisi parmi le diméthylformamide et la N-méthylpyrrolidone,
iii. est choisi parmi le phénol, la N-méthylpyrrolidone et la N-formylmorpholine,
iv. est une solution de chlorure de calcium dans de l'eau, ou est choisi parmi le diméthylsulfoxyde et l'éthylène glycol,
v. est l'acide sulfurique,
vi. est l'éthylène glycol,
vii. est le N,N-diméthylacétamide,
viii. est l'eau,
ix. est le diméthylsulfoxyde,
x. est choisi parmi l'éthylène glycol, la glycérine et le diméthylsulfoxyde,
xi. est l'eau,
xii. est choisi parmi le diméthylsulfoxyde, la glycérine et le diéthylène glycol, le 1-naphtol, l'hydroquinone et le diméthylformamide.

12. Procédé selon l'une quelconque des revendications précédentes, dans lequel A désigne le butane et B désigne le butène, le butadiène ou leurs mélanges, et l'agent d'extraction comprend de la N-méthylpyrrolidone.
